# EUROPÄISCHE PATENTSCHRIFT

(11) **EP 3 177 224 B1**
(45) Veröffentlichungstag und Bekanntmachung des Hinweises auf die Patenterteilung: **30.03.2022**
(21) Anmeldenummer: 15731025.1
(22) Anmeldetag: 18.06.2015
(51) Int. Cl.: B01F 13/00, B01F 15/02, A61B 17/00, A61B 17/88

(54) **MISCHVORRICHTUNG, INSBESONDERE AUSGEBILDET ZUM MISCHEN VON KNOCHENZEMENT**
MIXING DEVICE, IN PARTICULAR DESIGNED FOR MIXING BONE CEMENT
DISPOSITIF DE MÉLANGE, EN PARTICULIER CONÇU POUR LE MÉLANGE DE CIMENT OSSEUX

(30) Priorität: 04.08.2014 DE 102014111035
(43) Veröffentlichungstag der Anmeldung: 14.06.2017
(73) Patentinhaber: OSARTIS GmbH, 64839 Münster (DE)
(72) Erfinder: DEUSSER, Stefan, 63791 Karlstein (DE); ANDERS, Rike, 61440 Oberursel (DE)
(74) Vertreter: Blumbach · Zinngrebe Patentanwälte PartG mbB
(86) Internationale Anmeldenummer: PCT/EP2015/063731
(87) Internationale Veröffentlichungsnummer: WO 2016/020097

(56) Entgegenhaltungen:
- WO-A1-2013/179832
- WO-A2-2011/139921
- DE-B4-102009 013 211
- US-A- 5 569 210
- US-A- 5 569 210
- US-A1- 2013 253 465
- US-A1- 2013 253 465
- US-A1- 2014 021 076
- US-A1- 2014 021 076

## Beschreibung

### Gebiet der Erfindung

Die Erfindung betrifft eine Knochenzement-Mischvorrichtung.

### Hintergrund der Erfindung

Mischvorrichtungen, insbesondere solche zum Mischen von Komponenten von medizinischen Produkten, wie beispielsweise Knochenzement, sind bekannt.

Bei einem Zweikomponentensystem werden zwei Stoffe, insbesondere eine Flüssigkeit und ein Feststoff vor der Verwendung angemischt, sind für einen kurzen Zeitraum verarbeitbar und härten sodann aus.

Im medizinischen Bereich sind insbesondere Mischvorrichtungen für Methylmethacrylat basierten Knochenzement bekannt. Dabei wird in der Regel ein PMMA-Pulver mit einer Monomerkomponente vermischt, so dass eine aushärtbare Paste entsteht. Diese Paste lässt sich in der Regel sehr gut verarbeiten und dient insbesondere zum Einzementieren von Prothesen.

Das Deutsche Patent DE 10 2009 013 211 B4 zeigt eine derartige gattungsbildende Mischvorrichtung.

Die Monomerkomponente ist bei dieser Ausführungsvariante eines Knochenzementmischers an einem Griff des Mischsystems angeordnet. Vor der Verwendung wird ein Monomerfläschchen auf eine Nadel gedrückt. Aufgrund eines im Monomerfläschchen vorhandenen Gasvolumens sowie aufgrund des in der Mischkammer anliegenden Vakuums wird das Monomer in die Mischkammer geleitet.

Ein derartiges geschlossenes System ist einfach ausgestaltet und sicher in der Handhabung.

Nachteilig ist, dass leicht flüchtige Monomerkomponenten, wie Methylmethacrylat, je nach geltender Gesetzeslage nur in relativ kleinen Gebinden transportiert werden dürfen. Dies kann bei der eingangs genannten Mischvorrichtung die Menge, die mittels der Mischvorrichtung angerührt werden kann, für einige Anwendungen unerwünscht limitieren. Weiter ist vorstehendes System nur dazu ausgelegt, eine Flüssigkomponente und eine Festkomponente miteinander zu vermischen. Das System ist nicht dafür bestimmt, zwei in je einem Fläschchen vorhandene Monomerkomponenten, welche auch eine unterschiedliche Flüssigkeit umfassen können, miteinander zu mischen.

### Aufgabe der Erfindung

Der Erfindung liegt demgegenüber die Aufgabe zugrunde, eine Knochenzement-Mischvorrichtung einfacher und noch sicherer handhabbar auszugestalten.

### Zusammenfassung der Erfindung

Die Aufgabe der Erfindung wird durch eine Knochenzement-Mischvorrichtung nach dem unabhängigen Anspruch 1 gelöst.

Bevorzugte Ausführungsformen und Weiterbildungen der Erfindung sind dem Gegenstand der jeweiligen Unteransprüche zu entnehmen.

Die Knochenzement-Mischvorrichtung umfasst zumindest zwei mit einer Flüssigkeit gefüllte Ampullen. Als Ampulle kann jede geeignete Art von Behältnis verwendet werden. Insbesondere betrifft die Erfindung als Fläschchen mit einem einstechbaren Septum ausgebildete Glasampullen.

Gemäß der Erfindung sind zwei mit einer Flüssigkeit gefüllte Ampullen einstechbar und über die Nadeln ist die in den Ampullen vorhandene Flüssigkeit ausleitbar, wobei die Ampullen mittels eines Ampullenhalters gleichzeitig auf die Nadeln aufschiebbar sind.

Gemäß der Erfindung werden also zwei Ampullen gleichzeitig auf je eine Nadel aufgeschoben, so dass gleichzeitig die Flüssigkeit aus beiden Ampullen ausläuft.

Gegenüber einer Mischvorrichtung mit nur einer Ampulle hat dies einige Vorteile. So kann ggf. die Gebindegröße der einzelnen Ampullen reduziert werden.

Des Weiteren ist denkbar, mittels der Mischvorrichtung zwei verschiedene Flüssigkeiten zu mischen, indem diese beim oder nach dem Ausleiten zusammengebracht werden.

Weiter ist auch denkbar, je nach gewünschter Menge anzurührenden Knochenzements die Mischvorrichtung lediglich mit einer oder mit zwei oder mehreren Ampullen zu bestücken.

Vorzugsweise umfasst die Mischvorrichtung des Weiteren einen Mischraum, an welchem ein Unterdruck anlegbar ist. Die Flüssigkeit wird von den Ampullen in den Mischraum geleitet.

Beispielsweise kann dies über ein Röhrchen erfolgen, welches zugleich auch dafür verwendet werden kann, um den Knochenzement nach dem Anmischen aus der Mischkammer zu treiben, indem ein die Ampullen fassendes Gehäuse von der restlichen Mischvorrichtung abgetrennt wird oder um ein im Mischraum angeordnetes Paddel zu betätigen.

Vorzugsweise wird die Flüssigkeit aus den Ampullen durch ein in den Ampullen vorhandenes Gasvolumen ausgeleitet. Denkbar ist aber auch, die Ampullen zu belüften, beispielsweise über ein Membranventil oder die Nadeln derart dick auszugestalten, dass Luftblasen durch die Nadeln hochsteigen können und so ein Druckaustausch möglich ist.

Die Verwendung von zumindest zwei Ampullen bietet darüber hinaus den Vorteil, dass eine Entleerung schneller erfolgt.

Bei der Erfindung ist der Ampullenhalter in einer Kappe angeordnet, welche in ein Gehäuse, welches die Nadel umfasst, schiebbar ist.

Die Kappe kann insbesondere verschiebbar in einem Gehäuse verrastet sein, wobei der Ampullenhalter als separates Bauteil in die Kappe eingesetzt ist, etwa ebenfalls durch ein Verrasten.

Denkbar ist aber auch eine Variante, bei welcher der Ampullenhalter integraler Bestandteil der Kappe ist, da die Kappe mithin die Ampullen trägt.

Vorzugsweise umfasst die Mischvorrichtung einen Verteiler. Dieser weist Kanäle auf, welche mit den Nadeln verbunden sind. In dem Verteiler werden die Flüssigkeiten aus den zumindest zwei Ampullen zusammengeführt und können sodann über einen Kanal, etwa ein Rohr, ausgeleitet werden.

Zum Aufschieben der Ampullen kann ein Entriegelungsmittel betätigt werden. Dies bedeutet, der Ampullenhalter lässt sich erst in Richtung des Öffnungsorgans schieben, wenn gleichzeitig das Entriegelungsmittel betätigt ist. Im aufgeschobenen Zustand verrastet die Ampulle unlösbar.

Im Unterschied zum eingangs genannten Stand der Technik, bei welchem ein Sicherungsriegel entfernt wird, kann so das Entriegelungsmittel integraler Bestandteil des Gehäuses der Mischvorrichtung sein und es sind nicht durch das Entriegelungsmittel kleine Teile vorhanden, welche im Operationssaal eine potentielle Gefahr darstellen können.

Die zwei Ampullen sind in einer Kappe angeordnet, welche schiebbar in dem Gehäuse angeordnet ist und welche im nicht aufgeschobenen Zustand über das Gehäuse hinausragt.

Im aufgeschobenen und verrasteten Zustand ist anhand des zusammengeschobenen Gehäuses so für den Benutzer leicht zu erkennen, dass die Mischvorrichtung bereits benutzt wurde.

Durch das Verrasten ist ferner sichergestellt, dass die gesamte Flüssigkeit ausgeleitet wird, da der Benutzer das Ausleiten der Flüssigkeit nicht durch Hochziehen der Ampullen stoppen kann.

Die Nadeln können aus Kunststoff bestehen.

Die Kunststoffnadel ist insbesondere integraler Bestandteil eines Einsatzes, welcher in ein Gehäuse der Mischvorrichtung eingesetzt ist und welcher beispielsweise durch Verschweißen oder Verkleben mit dem Gehäuse der Mischvorrichtung verbunden ist.

Es hat sich herausgestellt, dass über einen derartigen Einsatz eine Kunststoffnadel als integraler Bestandteil des Einsatzes ausgebildet ist, welche ausreichend ist, um ein mit einem Septum versehenes Monomerfläschchen einzustechen. Dies hat erhebliche produktionstechnische Vorteile, da ein einziges Spritzgussteil verwendet werden kann, um Nadel und Einsatz auszubilden. Es muss insbesondere keine Kanüle aus Metall in die Spritzgussform eingesetzt werden, was die Herstellung schneller und preiswerter macht.

Der Einsatz ist insbesondere als zumindest zwei Nadeln umfassender Verteiler ausgebildet.

Dieser ist vorzugsweise zweistückig ausgebildet und besteht aus einer Nadeleinheit mit den beiden Kunststoffnadeln sowie einem Verteiler, welcher Kanäle umfasst, um die Flüssigkeiten aus beiden Nadeln zusammenzuführen.

Gemäß der Erfindung sind die Ampullen in einen Ampullenhalter eingesetzt, welcher in einer Kappe angeordnet ist, die ihrerseits verschiebbar in ein Gehäuse eingesetzt ist. Das Gehäuse umfasst zumindest ein Öffnungsorgan und einen Kanal, um die Flüssigkeit auszuleiten.

Das Gehäuse zur Aufnahme der Ampulle umfasst mithin drei wesentliche Hauptbestandteile, nämlich ein Gehäuse, welches auch als Hauptgehäuse betrachtet werden kann und welches beispielsweise ein Rohr zum Ausleiten der Flüssigkeit sowie das Öffnungsorgan aufweist.

Im Unterschied zum eingangs genannten Stand der Technik sind die Ampullen nicht unmittelbar in die Kappe eingesetzt, sondern werden von einem in die Kappe eingesetzten Ampullenhalter gehalten.

Dies ermöglicht zum einen eine vereinfachte Montage.

Zum anderen ist der Ampullenhalter vorzugsweise derartig ausgebildet, dass er zumindest ein Halteorgan umfasst, welches die Ampullen an einem Kragen in einer definierten Position hält.

In dem Halteorgan können die Ampullen insbesondere verrastet werden.

Durch das Halteorgan wird eine definierte Position der Ampullen zum Öffnungsorgan sichergestellt.

Dies beruht auf der Erkenntnis, dass beispielsweise bei bekannten Ampullen mit einem Septum die Toleranzen im Bereich des Septums bzw. der Kappe, mit welcher die Ampulle verschlossen ist, gering sind. Egal, ob nun das Halteorgan an der Kappe selbst oder einem Kragen der vorzugsweise aus Glas bestehenden Ampulle angreift, liegt immer eine hohe Maßhaltigkeit vor.

Die Länge von herkömmlichen Glasampullen unterliegt dagegen relativ hohen Toleranzen, welche nicht in jedem Fall sicher über die Konstruktion der Mischvorrichtung abgefangen werden können, wenn die Rückseite der Ampulle die Position der Ampulle in der Kappe definiert.

Bei einer bevorzugten Ausführungsform der Erfindung sind sowohl Ampullenhalter und Kappe verrastet als auch Kappe und Gehäuse verrastet.

Die Montage eines derartigen dreiteiligen Gehäuses für die Ampulle kann so auf sehr einfache Weise erfolgen.

### Beschreibung der Zeichnungen

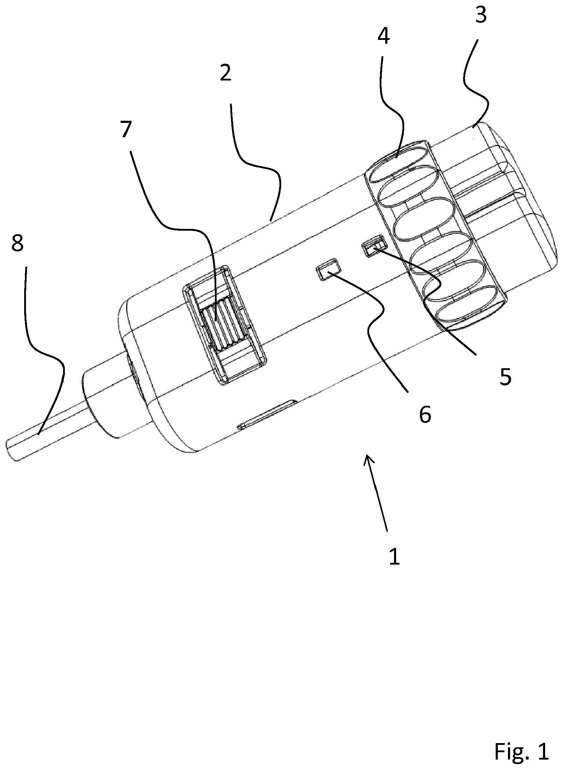

Der Gegenstand der Erfindung soll im folgenden Bezug nehmend auf ein in den Figuren Fig. 1 bis Fig. 14 dargestelltes Ausführungsbeispiel näher erläutert werden.

Fig. 1 zeigt eine perspektivische Ansicht einer Mischvorrichtung 1.

Die Mischvorrichtung 1 umfasst ein Gehäuse 2, welches in diesem Ausführungsbeispiel länglich mit im Wesentlichen rechteckigem Querschnitt und abgerundeten Ecken ausgebildet oder oval ist.

Die von der Kreiszylinderform abweichende Form des Gehäuses ermöglicht ein sicheres Greifen des Gehäuses insbesondere bei Verwendung des Gehäuses, in welchem die Ampullen angeordnet sind.

Zum Betätigen eines Mischers können die benötigten Schub- und Zugbewegungen bei gleichzeitiger Rotation des Mischpaddels leichter ausgeführt werden.

In das Gehäuse 2 eingeschoben ist eine Kappe 3, die zum Ausleiten der Flüssigkeit herabgedrückt wird.

Das Gehäuse 2 umfasst die Profilierung 4 mittels der dieses besser zu umgreifen ist. Eine derartige Profilierung kann insbesondere von Vorteil sein, wenn das hier dargestellte Gehäuse zum Antreiben eines Paddels zum Anmischen von Knochenzement in einer Mischkammer (nicht dargestellt) verwendet wird.

Zu erkennen ist ein als Taste oder Drücker ausgebildetes Entriegelungsmittel 7, welches bei dieser Ausführungsform der Erfindung spiegelsymmetrisch, also auch auf der anderen Seite angeordnet ist.

Weiter zu erkennen ist eine erste Ausnehmung 5 und eine zweite Ausnehmung 6 in dem Gehäuse 2.

In einem betätigten Zustand ist ein Rasthaken der Kappe 3 in der Ausnehmung 5 verrastet und verhindert, dass die Kappe 3 nach hinten ausgeschoben werden kann.

Betätigt nunmehr der Benutzer durch beidseitiges Drücken die Entriegelungsmittel 7, kann er die Kappe 3 vorschieben bzw. tiefer in das Gehäuse 2 drücken und ein Rasthaken an der Kappe 3 verrastet sodann in einem betätigten Zustand in der Ausnehmung 6.

Über das Rohr 8 tritt sodann die Flüssigkeit aus den in dem Gehäuse 2 angeordneten Ampullen aus.

Bei der Verwendung der Knochenzementmischvorrichtung ist das Rohr 8 mit einer Mischkammer (nicht dargestellt) verbunden, an welcher ein Vakuum anliegt und in welcher ein Feststoff mit der Flüssigkeit aus den Ampullen gemischt wird.

Das Gehäuse 2 kann dabei verwendet werden, um ein Mischpaddel anzutreiben. Anschließend kann das Gehäuse 2 an einer Sollbruchstelle (nicht dargestellt) des Rohres 8 abgetrennt werden, um den Knochenzement auszudrücken. Weiter kann das hier dargestellte Gehäuse 2, das zur Aufnahme der Monomerampulle ausgebildet ist, auch als Griff verwendet werden, um einen Kolben zu betätigen, um den Knochenzement auszudrücken (nicht dargestellt).

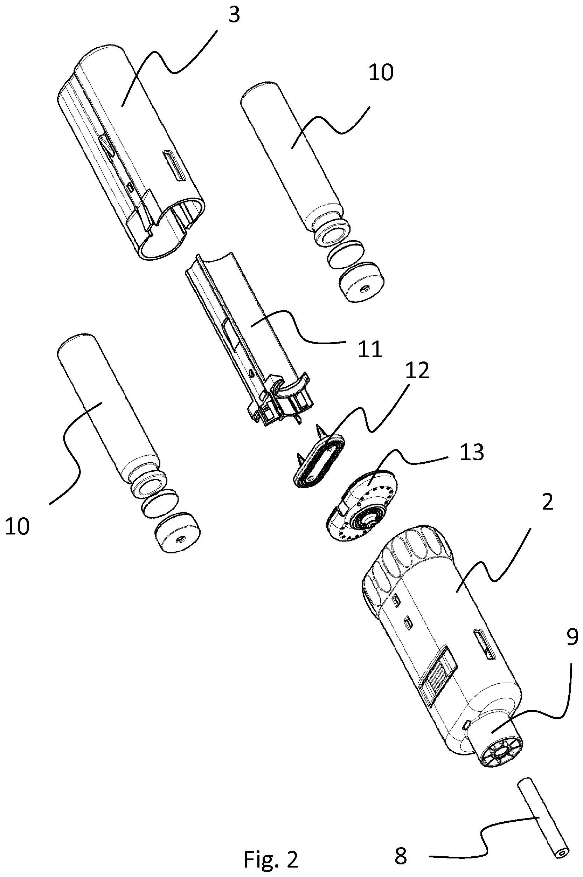

Fig. 2 zeigt die wesentlichen Bestandteile der in Fig. 1 dargestellten Mischvorrichtung in einer Explosionsdarstellung.

Zu erkennen ist das Gehäuse 2, an welchem an einem hier kreiszylindrisch ausgebildeten Fortsatz 9 das Rohr 8 zum Ausleiten der Flüssigkeit angebracht ist.

Weiter umfasst die Mischvorrichtung in diesem Ausführungsbeispiel zwei Monomerfläschchen 10, welche mit einem Septum verschlossen sind.

Die Ampullen 10 mit dem Monomer werden zunächst in einen Ampullenhalter 11 eingesetzt.

Der Ampullenhalter 11 wird sodann in die Kappe 3 eingeschoben, wo er verrastet.

Weiter zu erkennen ist eine Nadeleinheit 12, welche zwei Nadeln umfasst.

Die Nadeleinheit 12 ist im montierten Zustand mit einem Verteiler 13 verbunden, der seinerseits zusammen mit der Nadeleinheit 12 in das Gehäuse 2 eingesetzt ist und dort insbesondere verschweißt oder eingeklebt ist.

Wird nun, wie unter Bezugnahme auf Fig. 1 bereits beschrieben, die Kappe 3 heruntergedrückt, so werden die Ampullen 10 beide gleichzeitig auf die Nadeleinheit 12 aufgeschoben.

Etwa durch ein an dem Rohr 8 anliegendes Vakuum in Verbindung mit einem Gasvolumen in den Ampullen 10 läuft nunmehr die Flüssigkeit durch die Nadeln der Nadeleinheit 12 und den Verteiler 13 in das Rohr 8 und tritt beispielsweise in eine Mischkammer aus.

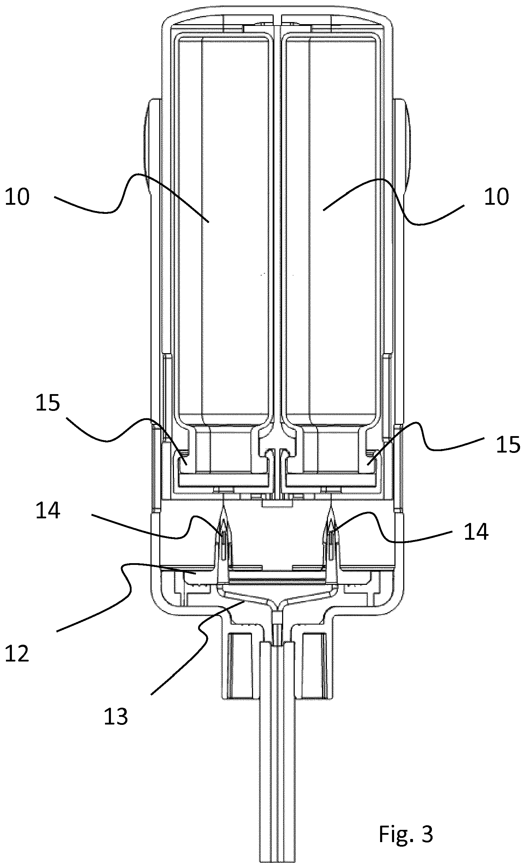

Fig. 3 zeigt eine Schnittdarstellung einer Mischvorrichtung im vormontierten, aber nicht betätigten Zustand.

Zu erkennen sind die beiden parallel angeordneten Ampullen 10, welche an einem Kragen 15 in einer definierten Position gehalten werden.

Weiter zu erkennen sind die Nadeln 14 der Nadeleinheit 12. Die Nadeleinheit 12 ist mit dem Verteiler 13 verbunden, welcher Kanäle aufweist, in denen die Flüssigkeit aus beiden Ampullen 10 zusammengeführt wird.

Das erfindungsgemäße System kann mithin auch verwendet werden, um zwei Flüssigkeiten miteinander zu mischen.

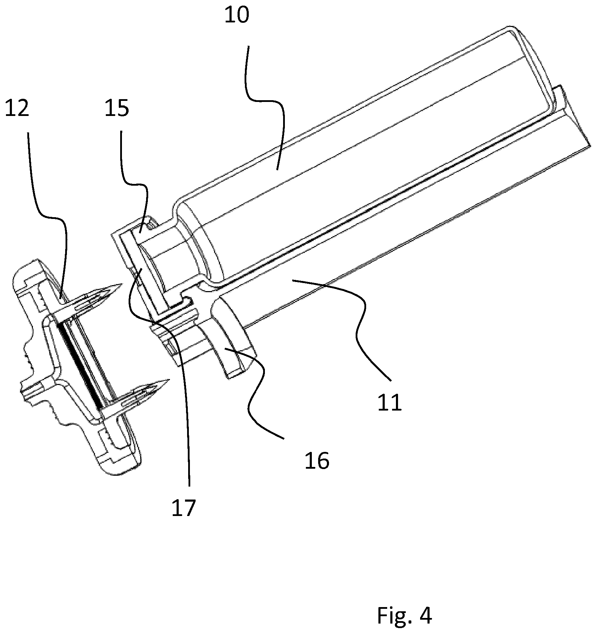

Fig. 4 zeigt eine perspektivische Schnittansicht, bei welcher das Gehäuse, die Gehäusekappe und eine Ampulle ausgeblendet sind.

Zu erkennen ist, dass die Ampulle 10 mit einem Kragen 15 in einem Halteorgan 16 eines Ampullenhalters sitzt.

Das Septum 17 ist so direkt gegenüber der Nadel der Nadeleinheit 12 angeordnet.

Die Verwendung eines Ampullenhalters ermöglicht neben der besseren definierten Position der Ampulle 10 eine verbesserte Montage.

Vorzugsweise wird die Ampulle 10 in dem Halteorgan 16 verrastet.

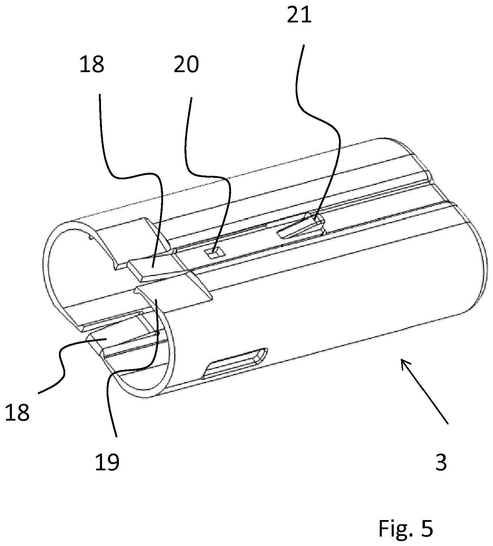

Fig. 5 zeigt eine perspektivische Ansicht der Kappe.

Die Kappe ist zum Einschieben in das Gehäuse (2 in Fig. 1) ausgebildet.

Die Kappe umfasst Sperrriegel 18, welche gegenüber einer angrenzenden Gehäusewand 19 hervorstehen.

Die Sperrriegel 18 können durch Betätigung der Entriegelungsmittel (7 in Fig. 1) hereingedrückt werden, um die Kappe und damit die in die Kappe eingesetzten Ampullen zum Verschieben freizugeben.

Weiter umfasst die Kappe einen Rasthaken 21, welcher im vormontierten, aber noch nicht betätigten Zustand in der oberen Ausnehmung des Gehäuses (5 in Fig. 1) verrastet und welcher im betätigten Zustand in der unteren Ausnehmung (6 in Fig. 1) des Gehäuses verrastet.

Eine weitere Ausnehmung 20 an der Kappe dient dem Verrasten von Kappe und Ampullenhalter (11 in Fig. 2).

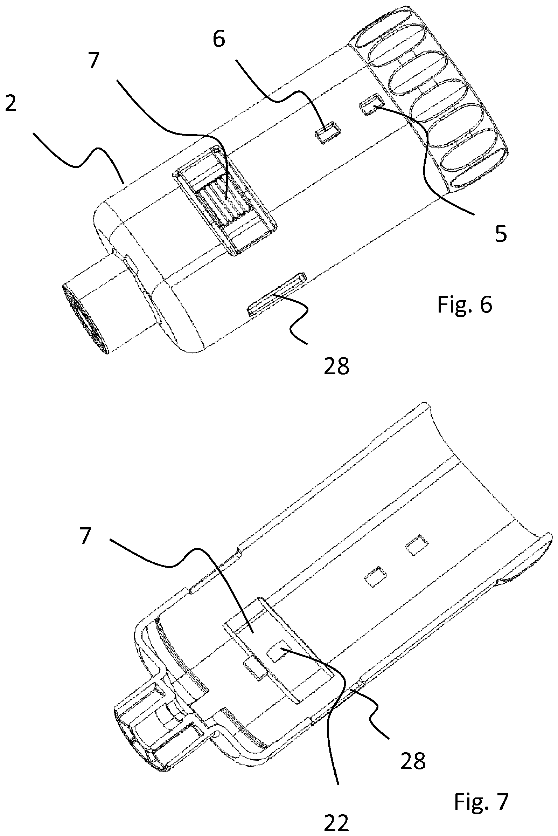

Fig. 6 zeigt eine weitere perspektivische Ansicht des Gehäuses 2. Zu erkennen ist insbesondere die obere Ausnehmung 5 zum Verrasten der in Fig. 5 dargestellten Kappe im vormontierten Zustand sowie die Ausnehmung 6 zum Verrasten im betätigten Zustand.

Das Verrasten der Kappe stellt sicher, dass der Benutzer ohne die Verwendung von Werkzeug die Kappe weder abnehmen kann noch im betätigten Zustand diese wieder nach hinten ziehen kann.

Weiter zu erkennen sind die als Drücker ausgebildeten Entriegelungsmittel 7, welche als integraler Bestandteil des Gehäuses 2 ausgebildet sind. Hierzu umfasst das Gehäuse 2 randseitig der Entriegelungsmittel 7 Schlitze, so dass das profilierte Entriegelungsmittel 7 wie ein Knopf herabgedrückt werden kann.

Fig. 7 zeigt eine perspektivische Schnittansicht des Gehäuses. Zu erkennen ist insbesondere die Unterseite des Entriegelungsmittels 7. Diese weist einen Fortsatz 22 auf, welcher im montierten Zustand beim Drücken des Entriegelungsmittels 7 die Sperrriegel (18 in Fig. 5) betätigt.

Das Gehäuse weist des Weiteren randseitige Öffnungen 28 auf, durch welche beispielsweise der Inhalt der Ampullen für den Benutzer sichtbar ist.

Fig. 8 und Fig. 9 zeigen Schnittansichten, anhand welcher die Funktion der verschiedenen Rast- und Sperrmittel erläutert werden soll.

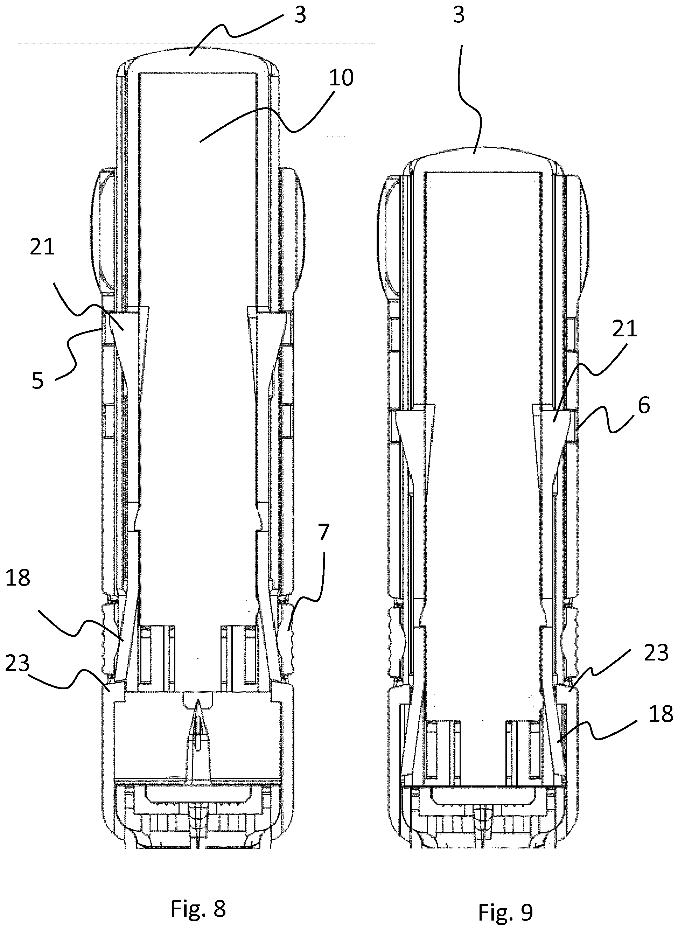

Fig. 8 zeigt die Mischvorrichtung im vormontierten, aber nicht betätigten Zustand.

Die Ampullen 10 sitzen in der Kappe 3.

Die Kappe weist Rasthaken auf, die in einer oberen Ausnehmung 5 des Gehäuses verrastet sind und die verhindern, dass die Kappe zurückgezogen werden kann.

Die vordere Position der Kappe 3 und damit der Ampulle 10 wird durch die Sperrriegel 18 begrenzt, welche sich nach vorne hin aufspreizen und auf einem Kragen 23 des Gehäuses aufliegen.

Betätigt der Benutzer nunmehr die Entriegelungsmittel 7 durch Drücken, werden die Sperrriegel 18 nach innen gedrückt und lassen sich beim Drücken der Kappe an dem Kragen vorbei nach vorne drücken.

Die Ampulle wird sodann mit dem Septum auf die Nadel gedrückt.

Fig. 9 zeigt eine Schnittansicht im montierten Zustand.

Die Rasthaken 21 sind nunmehr in der tieferliegenden Ausnehmung 6 des Gehäuses verrastet, so dass die Kappe 3 auch nicht mehr zurückgeschoben werden kann.

Die Sperrriegel 18 sind am Kragen 23 vorbeigeglitten.

Die vordere Endposition kann beispielsweise durch Aufliegen der Ampulle auf der Nadeleinheit definiert sein.

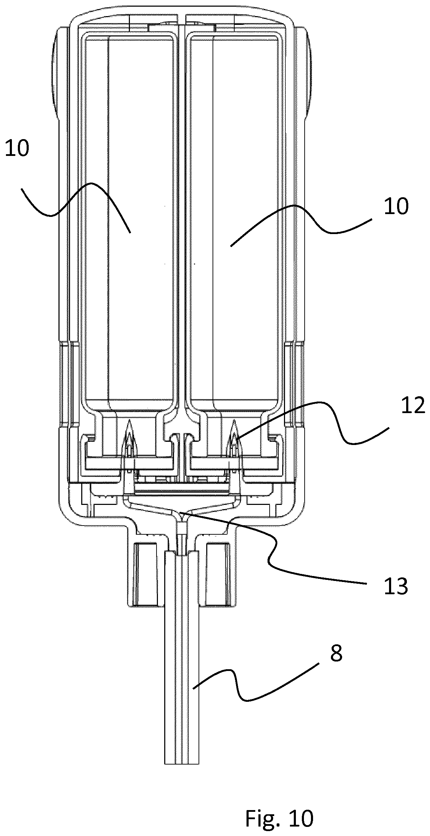

Fig. 10 zeigt eine Schnittansicht der Mischvorrichtung im betätigten Zustand.

Die Ampullen 10 sind mit ihrem Septum auf die Nadeleinheit 12 aufgeschoben. Von den Nadeln der Nadeleinheit 12 laufen die Flüssigkeiten der Ampullen 10 in den Verteiler 13 und werden dort zusammengeführt, um über das Rohr 8 entnommen zu werden.

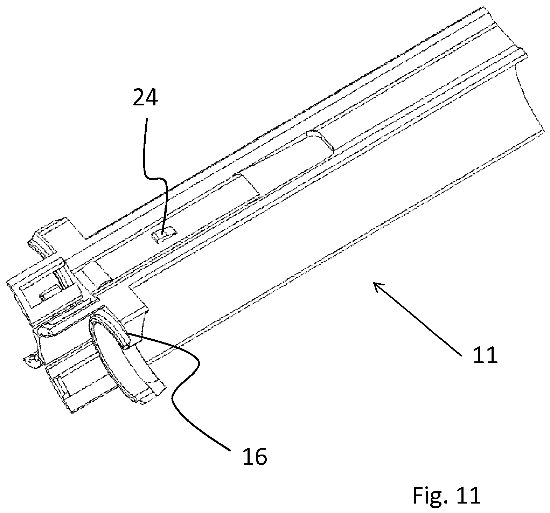

Fig. 11 zeigt eine perspektivische Ansicht des Ampullenhalters 11.

Zu erkennen sind insbesondere die sich von dem länglichen Träger seitlich weg erstreckenden Halteorgane 16, welche ringsegmentförmig ausgebildet sind und in welche die Ampullen eingerastet werden können.

Der Halter weist ferner einen Rasthaken 24 auf, mit dem der Halter zusammen mit den Ampullen in die Kappe geschoben wird.

Der Rasthaken verrastet den Halter zusammen mit den Ampullen sodann in einer Ausnehmung der Kappe (20 in Fig. 5) .

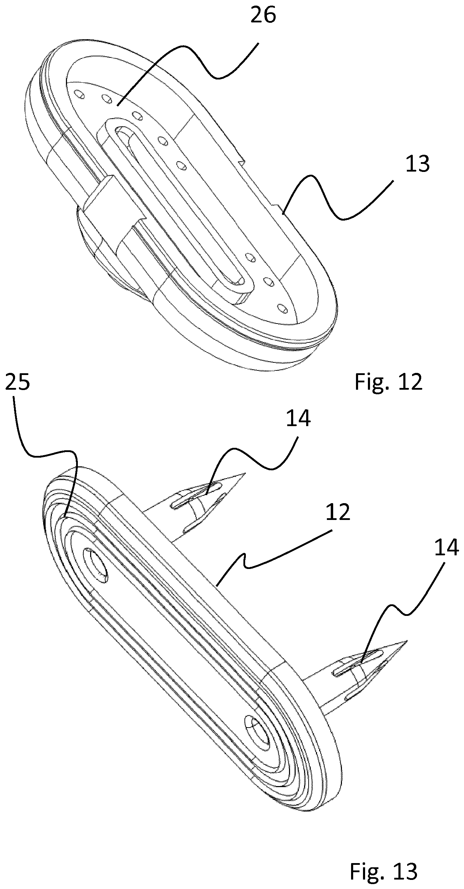

Fig. 12 und Fig. 13 zeigen perspektivische Ansichten von Verteiler 13 und Nadeleinheit 12.

Verteiler 13 und Nadeleinheit 12 sind in diesem Ausführungsbeispiel beide aus Kunststoff ausgebildet.

Der Verteiler 13 weist eine Wanne 26 auf, in welche die Nadeleinheit 12 eingesetzt wird. Die Nadeleinheit 12 umfasst auf ihrer Unterseite eine Profilierung 25, etwa in Form von umlaufenden Stegen, welche dem Verschweißen mittels Ultraschall dient.

Die Nadeln 14 sind zusammen mit der restlichen Nadeleinheit 12 als einstückiges Spritzgussbauteil ausgebildet.

Die Nadeln 14 weisen in diesem Ausführungsbeispiel eine Spitze sowie sich hinter der Spitze erstreckende seitliche Schlitze auf. So wird trotz der Verwendung eines Kunststoffbauteils ein hoher Durchfluss sichergestellt.

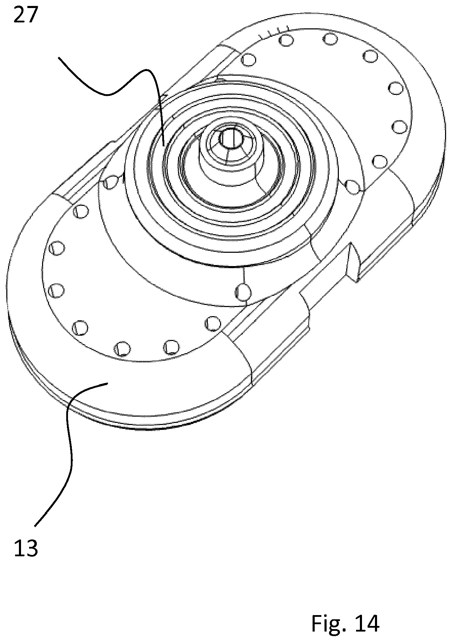

Fig. 14 zeigt eine perspektivische Ansicht der Unterseite des Verteilers 13. Auch die Unterseite des Verteilers 13 weist eine Profilierung 27 auf, die dem Verschweißen des Verteilers 13 mit dem Gehäuse der Mischvorrichtung dient.

### Bezugszeichenliste

- 1: Mischvorrichtung
- 2: Gehäuse
- 3: Kappe
- 4: Profilierung
- 5: Ausnehmung
- 6: Ausnehmung
- 7: Entriegelungsmittel
- 8: Rohr
- 9: Fortsatz
- 10: Ampulle
- 11: Ampullenhalter
- 12: Nadeleinheit
- 13: Verteiler
- 14: Nadel
- 15: Kragen
- 16: Halteorgan
- 17: Septum
- 18: Sperrriegel
- 19: Gehäusewand
- 20: Ausnehmung
- 21: Rasthaken
- 22: Fortsatz
- 23: Kragen
- 24: Rasthaken
- 25: Profilierung
- 26: Wanne
- 27: Profilierung
- 28: Öffnung

## Patentansprüche

1. Knochenzement-Mischvorrichtung (1), umfassend zumindest zwei Nadeln (14), einen Ampullenhalter (11), ein Gehäuse (2), eine Kappe (3) und zwei mit einer Flüssigkeit gefüllte Ampullen (10), welche mit jeweils einer Nadel (14) einstechbar sind und über die Nadeln (14) die Flüssigkeit aus den Ampullen (10) ausleitbar ist, wobei die Ampullen mittels des Ampullenhalters (11) gleichzeitig auf die Nadeln (14) aufschiebbar sind, wobei die Ampullen (10) in den Ampullenhalter (11) eingesetzt sind, welcher in der Kappe (3) angeordnet ist, die verschiebbar in das Gehäuse (2) eingesetzt ist.

2. Mischvorrichtung nach dem vorstehenden Anspruch, **dadurch gekennzeichnet, dass** die Mischvorrichtung einen Verteiler (13) umfasst, in welchem die Flüssigkeit aus den Ampullen (10) vor dem Ausleiten mischbar ist.

3. Mischvorrichtung (1) nach einem der vorstehenden Ansprüche, **dadurch gekennzeichnet, dass** zum Aufschieben der Ampullen (10) ein Entriegelungsmittel gedrückt werden muss, wobei in einem aufgeschobenen Zustand die Ampullen (10) unlösbar verrasten.

4. Mischvorrichtung nach dem vorstehenden Anspruch, **dadurch gekennzeichnet, dass** die Ampullen (10) in einer Kappe (3) angeordnet ist, welche schiebbar in einem Gehäuse (2) angeordnet ist und welche zumindest im nicht aufgeschobenen Zustand über das Gehäuse (2) hinausragt.

5. Mischvorrichtung (1) nach einem der vorstehenden Ansprüche, **dadurch gekennzeichnet, dass** die Nadeln (14) aus Kunststoff bestehen.

6. Mischvorrichtung nach dem vorstehenden Anspruch, **dadurch gekennzeichnet, dass** die Nadeln (14) Teil eines mit einem Gehäuse (2) der Mischvorrichtung verbundenen Einsatzes ist.

7. Mischvorrichtung nach dem vorstehenden Anspruch, **dadurch gekennzeichnet, dass** der Einsatz als mindestens zwei Nadeln (14) umfassender Verteiler ausgebildet ist.

8. Mischvorrichtung nach einem der vorstehenden Ansprüche, **dadurch gekennzeichnet, dass** der Ampullenhalter (11) in der Kappe (3) verrastet ist und/oder dass die Kappe in dem Gehäuse (2) verrastet ist und/oder dass der Ampullenhalter zumindest ein Halteorgan (16) aufweist, welches die Ampulle (10) an einem Kragen (15) hält.

## Claims

1. A bone cement mixing device (1), comprising at least two needles (14), an ampoule holder (11), a housing (2), a cap (3) and two ampoules (10) filled with a liquid, which can be perforated by a respective needle (14), and wherein the liquid is dischargeable from the ampoules (10) through the needles (14);
wherein the ampoules can be slidingly engaged with the needles (14) simultaneously, by means of the ampoule holder (11);
wherein the ampoules are placed in the ampoule holder (11) which is arranged in the cap (3) which is slidably accommodated in the housing (2).

2. The mixing device according to the preceding claim, **characterised in that** the mixing device comprises a manifold (13) in which the liquid from the ampoules (10) can be mixed before being discharged.

3. The mixing device (1) according to any one of the preceding claims, **characterised in that** an unlocking means has to be pressed for slidingly engaging the ampoules (10), wherein in their engaged position the ampoules (10) are latched non-detachably.

4. The mixing device according to the preceding claim, **characterised in that** the ampoules (10) are arranged in a cap (3) which is slidably accommodated in a housing (2) and which projects beyond the housing (2), at least in the non-engaged position.

5. The mixing device (1) according to any one of the preceding claims, **characterised in that** the needles (14) are made of plastics material.

6. The mixing device according to the preceding claim, **characterised in that** the needles (14) form part of an insert that is connected to a housing (2) of the mixing device.

7. The mixing device according to the preceding claim, **characterised in that** the insert is provided in the form of a manifold comprising at least two needles (14).

8. The mixing device according to the preceding claim, **characterised in that** the ampoule holder (11) is latched in the cap (3); and/or that the cap is latched in the housing (2); and/or that the ampoule holder has at least one retaining member (16) which holds the ampoule (10) at a collar (15).

## Revendications

1. Dispositif de mélange de ciment osseux (1), comprenant au moins deux aiguilles (14), un support d'ampoules (11), un boîtier (2), un capuchon (3) et deux ampoules (10) remplies avec un liquide, dans lesquelles peut être piquée respectivement une aiguille (14) et où le liquide peut être évacué des ampoules (10) par l'intermédiaire des aiguilles (14), les ampoules pouvant être poussées en même temps sur les aiguilles (14) à l'aide du support d'ampoules (11), les ampoules (10) étant insérées dans le support d'ampoules (11) qui est disposé dans le capuchon (3), lequel est inséré dans le boîtier (2) avec possibilité de déplacement.

2. Dispositif de mélange selon la revendication précédente, **caractérisé en ce que** le dispositif de mélange comprend un distributeur (13) dans lequel le liquide provenant des ampoules (10) peut être mélangé avant d'être évacué.

3. Dispositif de mélange (1) selon l'une des revendications précédentes, **caractérisé en ce que** pour pousser les ampoules (10), il faut presser un moyen de déverrouillage, les ampoules (10) étant encliquetées de façon inamovible à l'état poussé.

4. Dispositif de mélange selon la revendication précédente, **caractérisé en ce que** les ampoules (10) sont placées dans un capuchon (3) qui est disposé de manière coulissante dans un boîtier (2) et qui dépasse par rapport au boîtier (2) au moins à l'état non poussé.

5. Dispositif de mélange (1) selon l'une des revendications précédentes, **caractérisé en ce que** les aiguilles (14) sont en matière synthétique.

6. Dispositif de mélange (1) selon la revendication précédente, **caractérisé en ce que** les aiguilles (14) font partie d'un insert qui est relié au boîtier (2) du dispositif de mélange.

7. Dispositif de mélange selon la revendication précédente, **caractérisé en ce que** l'insert est réalisé sous la forme d'un distributeur comprenant au moins deux aiguilles (14).

8. Dispositif de mélange selon l'une des revendications précédentes, **caractérisé en ce que** le support d'ampoules (11) est encliqueté dans le capuchon (3) et/ou **en ce que** le capuchon est encliqueté dans le boîtier (2) et/ou **en ce que** le support d'ampoules présente au moins un organe de maintien (16) qui maintient l'ampoule (10) au niveau d'un collet (15).
